# EUROPÄISCHE PATENTANMELDUNG

(11) **EP 3 889 939 A1**
(43) Veröffentlichungstag der Anmeldung: **06.10.2021**
(21) Anmeldenummer: 21164509.8
(22) Anmeldetag: 24.03.2021
(51) Int. Cl.: G08C 17/02

(54) **KOPPLUNG VON FUSSSCHALTER UND MEDIZINTECHNISCHEM GERÄT**

(30) Priorität: 30.03.2020 DE 102020108701
(71) Anmelder: Olympus Winter & Ibe GmbH, 22045 Hamburg (DE)
(72) Erfinder: Rezaie, Hamid, 22119 Hamburg (DE)
(74) Vertreter: Glawe, Delfs, Moll

(57) **Zusammenfassung**

Die Erfindung betrifft ein System (1) umfassend einen Fußschalter (10) zur Steuerung eines medizintechnischen Geräts (2) und einen Receiver (20)für das zu steuernde medizintechnische Gerät (2) , sowie ein Verfahren zur Kopplung von Fußschalter (10) und Receiver (20).

Dabei sind Fußschalter (10) und Receiver (20) zur drahtlosen Übermittlung von eindeutig zuordenbaren Steuerungssignalen (90) nach erfolgter Kopplung wenigstens vom Fußschalter (10) zum Receiver (20) ausgebildet. Eine der Komponenten (20, 10) umfasst ein NFC-Lesemodul (21) zum Auslesen eines Transponders (11) im Nahbereich (22) und die andere Komponente (10, 20) umfasst einen vom NFC-Lesemodul (21) auslesbaren Transponder (11), wobei die Komponente (20, 10) umfassend das NFC-Lesemodul (12) dazu ausgebildet ist, die Kopplung von Fußschalter (10) und Receiver (20) auf Basis von vom Transponder (11) ausgelesenen Informationen wenigstens zu initiieren.

Das Verfahren basiert auf dem erfindungsgemäßen System und umfasst die Schritte:
a) Initiieren der Kopplung zwischen Fußschalter (10) und Receiver (20) durch Heranführen des Fußschalters (10) an den Receiver (20), sodass sich der Transponder (11) der einen Komponente (10, 20) im Nahbereich des NFC-Lesemoduls (21) der anderen Komponente (20, 10) befindet; und
b) Abwarten bis die zur Kopplung erforderlichen Informationen ausgetauscht sind und die Kopplung abgeschlossen ist.

## Beschreibung

Die Erfindung betrifft ein System umfassend einen Fußschalter zur Steuerung eines medizintechnischen Geräts und einen an dem zu steuernden medizintechnischen Gerät angeschlossenen Receiver, sowie ein Verfahren zur Kopplung von Fußschalter und Receiver.

In der Medizintechnik sind Fußschalter zur Steuerung von medizintechnischen Geräten gängig. Durch Fußschalter ist es bspw. möglich, den Aktivierungszustand eines handgeführten Instruments zu steuern, ohne dass dazu die Führungshand selbst oder auch nur ein Finger davon bewegt werden müsste. Das medizinische Personal kann sich so allein auf die Führung des Instruments per Hand konzentrieren und den Aktivierungszustand des Instruments losgelöst davon per Fuß steuern. Der Aktivierungszustand muss dabei nicht auf das Ein- und Ausschalten einer bestimmten Funktion des handgeführten Instruments beschränkt sein, sondern kann auch eine stufenlose Einstellbarkeit umfassen. Beispiele für medizinische Geräte mit entsprechender Fußsteuerung sind Zahnarztinstrumente, wie Bohrer, HF-Generatoren für die Hochfrequenz-Chirurgie, Ultraschall-Generator für Ultraschallskalpelle und/oder Lasersysteme.

Fußschalter können mit dem zu steuernden medizinischen Gerät per Kabel verbunden sein. Um die Bewegungsfreiheit des medizinischen Personals bspw. in einem Operationssaal nicht einzuschränken oder zu behindern, sind aber auch Fußschalter bekannt, die drahtlos Steuerungssignale an das zu steuernde medizinische Gerät übertragen. Ein geeigneter Empfänger für die Steuerungssignale muss dann entweder unmittelbar in das medizinische Gerät integriert sein, oder es ist ein geeigneter externer Empfänger an dem Signaleingang, an dem bspw. auch ein kabelgebundener Fußschalter angeschlossen werden kann, vorzusehen.

Um sicherzustellen, dass mit einem Fußschalter tatsächlich nur ein bestimmtes medizinisches Gerät gesteuert wird, ist es zwingend erforderlich, den Fußschalter eindeutig mit einem Empfänger derart zu koppeln, dass die vom Fußschalter ausgesendeten Steuerungssignale ausschließlich von dem gekoppelten Empfänger verarbeitet werden. Dazu enthalten die Steuerungssignale regelmäßig eine eindeutige Codierung, welche die Steuerungssignale eindeutig als von einem bestimmten Fußschalter kommend identifiziert. Entsprechendes ist bspw. in EP 1 629 786 A1 beschrieben.

Die freie Kopplung eines Fußschalters mit einem bestimmten Empfänger, unabhängig davon, ob dieser als externe Einheit ausgebildet oder in ein zu steuerndes medizinisches Gerät integriert ist, ist im Stand der Technik häufig nicht vorgesehen oder nur sehr aufwendig möglich, sodass im Regelfall grundsätzlich eine 1-zu-1-Zuordnung eines Fußschalters mit einem dadurch zu steuernden medizinischen Gerät, zumindest aber mit einer bestimmten Empfängereinheit besteht.

Aufgabe der vorliegenden Erfindung ist es, ein System und ein Verfahren zu schaffen, bei denen ein Fußschalter auf einfache Weise mit einem Empfänger zur Steuerung eines medizinischen Geräts gekoppelt werden kann.

Gelöst wird diese Aufgabe durch ein System gemäß dem Hauptanspruch sowie ein Verfahren gemäß dem nebengeordneten Anspruch. Vorteilhafte Weiterbildungen sind Gegenstand der abhängigen Ansprüche.

Demnach betrifft die Erfindung ein System umfassend einen Fußschalter zur Steuerung eines medizintechnischen Geräts und einen an dem zu steuernden medizintechnischen Gerät angeschlossenen Receiver als Komponenten, wobei Fußschalter und Receiver zur drahtlosen Übermittlung von eindeutig zuordenbaren Steuerungssignalen nach erfolgter Kopplung wenigstens vom Fußschalter zum Receiver ausgebildet sind, wobei eine der Komponenten ein NFC-Lesemodul zum Auslesen eines Transponders im Nahbereich und die andere Komponente einen vom NFC-Lesemodul auslesbaren Transponder umfasst, und wobei die Komponente umfassend das NFC-Lesemodul dazu ausgebildet ist, die Kopplung von Fußschalter und Receiver auf Basis von vom Transponder ausgelesenen Informationen wenigstens zu initiieren.

Die Erfindung betrifft weiterhin ein Verfahren zur Kopplung eines Fußschalters zur Steuerung eines medizintechnischen Geräts und einen Receiver für das zu steuernde medizintechnische Gerät eines erfindungsgemäßen Systems, mit den Schritten:
a) Initiieren der Kopplung zwischen Fußschalter und Receiver durch Heranführen des Fußschalters an den Receiver, sodass sich der Transponder der einen Komponente im Nahbereich des NFC-Lesemoduls der anderen Komponente befindet; und
b) Abwarten bis die zur Kopplung erforderlichen Informationen ausgetauscht sind und die Kopplung abgeschlossen ist.

Zunächst werden einige in Zusammenhang mit der Erfindung verwendete Begriffe erläutert.

Mit "Koppeln" ist eine initiale Verbindungsaufnahme zwischen zwei Komponenten bezeichnet, bei der Informationen ausgetauscht werden, die für den Aufbau einer 1-zu-1-Verbindung zwischen den beiden Komponenten erforderlich sind. Die zur Kopplung ausgetauschten Informationen können auch Daten enthalten, um die nachfolgende Verbindung zwischen den beiden Komponenten durch Signierung oder Verschlüsselung des Datenaustausches zu sichern.

Mit "Nahfeldkommunikation" (Near Field Communication; NFC) ist eine drahtlose Datenkommunikation bezeichnet, deren Reichweite technisch auf kurze Strecken von wenigen Zentimetern limitiert ist. So ist bspw. bei dem gleichnamigen internationalen NFC-Übertragungsstandard der kontaktlose Austausch von Daten durch elektromagnetische Induktion zwischen zwei Spulen unter Rückgriff auf die RFID-Technologie, insbesondere mit passiven Transpondern, vorgesehen. Aufgrund der kurzen Reichweite der Datenübertragung ist der Abgriff von per NFC ausgetauschten Daten durch Dritte kaum möglich.

Bei einem "NFC-Lesemodul" handelt es sich um ein Modul zum Auslesen von Informationen von einem geeigneten Transponder, der sich im Nahbereich zum Lesemodul befindet, damit die Nahfeldkommunikation, auf die das Lesemodul beschränkt ist, erfolgen kann.

Steuerungssignale sind "eindeutig zuordenbar", wenn sie eine Charakteristik aufweisen, die einem Empfänger der Signale ermöglicht festzustellen, dass ein Steuerungssignal von einem bestimmten Sender oder zumindest von dem gleichen Sender, wie ein anderes Steuerungssignal, ausgesendet wurde. Im Falle von drahtlos übertragenen Steuerungssignalen kann es sich bei dieser Charakteristik um Eigenschaften der Drahtlosübertragung handeln (wie bspw. Frequenz, Modulation, etc.). Es ist aber bevorzugt, wenn das Steuerungssignal als Charakteristik selbst eine Identifikation aufweist, bspw. in Form einer den Sender eindeutig kennzeichnenden Identifikationsnummer und/oder -zeichenkette oder durch elektronische Signatur des übertragenen Steuerungssignals mit einem eindeutig zuordenbaren Schlüssel.

Der Erfindung hat erkannt, dass ein praktisch beliebiges Koppeln eines Fußschalters mit einem an ein medizinisches Gerät angebundenen Empfänger einfach und insbesondere sicher ist, wenn die Kopplung durch eine Nahfeldkommunikation zumindest initiiert wird. Da eine entsprechende Nahfeldkommunikation nur im Nahbereich möglich ist (d. h. die beiden zu koppelnden Geräte müssen für die Kopplung physisch nah beieinander angeordnet sein), ist zum Einen grundsätzlich sichergestellt, dass der Fußschalter auch tatsächlich mit dem gewünschten Empfänger und nicht etwa einem weiter entfernt angeordneten Empfänger gekoppelt wird, zum anderen ist ein unerwünschtes Abgreifen der zumindest für die Initiierung der Kopplung ausgetauschten Informationen durch Dritte aufgrund der inhärenten Eigenschaften der Nahfeldkommunikation praktisch ausgeschlossen.

Um die Kopplung zumindest zu initiieren, ist wenigstens eine der beiden Komponenten eines erfindungsgemäßen Systems umfassend einen Fußschalter und einen Receiver mit einem NFC-Lesemodul ausgestattet, mit dem ein geeigneter Transponder an der anderen Komponente ausgelesen werden kann, sofern sich dieser Transponder im Nahbereich des NFC-Lesemoduls befindet.

Es ist auch möglich, dass beide Komponenten jeweils ein NFC-Lesemodul und einen Transponder aufweisen. In diesem Fall ist ein wechselseitiges Auslesen von Informationen des Transponders der jeweils anderen Komponente möglich, die dann dazu genutzt werden können, die Kopplung wenigstens zu initiieren.

Unabhängig davon, ob lediglich eine oder beide Komponenten über ein NFC-Lesemodul verfügen, sind die vom Transponder der jeweils anderen Komponente ausgelesenen Informationen wenigstens ausreichend, um eine Kopplung zu initiieren. Es ist aber auch möglich, dass die dabei ausgetauschten Informationen ausreichend sind, die Kopplung der Komponenten unmittelbar abzuschließen.

Ist es für die eindeutige Zuordnung von Steuerungssignalen eines Fußschalters durch einen Receiver bspw. ausreichend, dass dem Receiver dessen eindeutige Identifikation und/oder den öffentlichen Signaturschlüssel bekannt ist und sind diese Informationen vollständig auf dem durch das NFC-Lesemodul auszulesenden Transponder enthalten, kann die Kopplung bereits nach dem Auslesen der Informationen vom Transponder und Verarbeiten allein aufseiten des Receivers vollständig abgeschlossen werden. Die Informationen auf dem Transponder können auch detailliertere Daten zu der Komponente, an der der Transponder angeordnet ist, enthalten, anhand derer die andere Komponente die Kompatibilität überprüfen und die Kopplung bei negativem Ergebnis unterbinden kann.

Alternativ dazu können die genannten Informationen genutzt werden, um eine Datenverbindung zwischen Fußschalter und Receiver aufzubauen, über die z. B. zunächst ein sog. Handshake durchgeführt wird, bei der evtl. Verschlüsselungsalgorithmen aushandelt oder die wechselseitige Kompatibilität von Fußschalter und Receiver überprüft wird, bevor die beiden Komponenten tatsächlich miteinander gekoppelt werden.

Selbst wenn eine Kopplung allein aufgrund der vom Transponder ausgelesenen Informationen erfolgt, kann durch eine entsprechende Datenverbindung die Kopplung nochmals verifiziert werden.

Ist nur ein NFC-Lesemodul zum Auslesen eines Transponders im Nahbereich vorgesehen, ist bevorzugt, wenn dieses am Receiver angeordnet ist und der Fußschalter vom NFC-Lesemodul auslesbaren Transponder aufweist. Eine entsprechende Anordnung ist insbesondere für die oben beschriebene vollständige Kopplung allein auf Basis von aus dem Transponder ausgelesenen Informationen vorteilhaft.

Der Nahbereich, in dem das NFC-Lesemodul einen Transponder auslesen kann, umfasst vorzugsweise einen maximalen Abstand zwischen Transponder und Lesegerät von 10 cm, weiter vorzugsweise von 5 cm. Durch einen entsprechend kurzen Abstand ist eine eindeutige und abhörsichere Kopplung zwischen Fußschalter und Receiver möglich.

Um zu verhindern, dass unbeabsichtigt eine Kopplung stattfindet, ist bevorzugt, wenn das oder die NFC-Lesemodule im Grundzustand deaktiviert und zur Kopplung aktivierbar sind. Ist eine Kopplung gewünscht, kann ein NFC-Lesemodul nur zeitweise - nämlich für die tatsächliche Kopplung - aktiviert werden. Eine unbeabsichtigte Kopplung eines Fußschalters durch versehentliches Heranführen an ein NFC-Lesemodul kann so vermieden werden.

Der Receiver kann grundsätzlich an einer dafür geeigneten Schnittstelle des medizinischen Geräts angeschlossen sein, bspw. an eine für einen kabelgebundenen Fußschalter vorgesehene Schnittstelle. Es ist bevorzugt, wenn der Receiver und/oder ein ggf. dem Receiver zugeordnetes NFC-Lesemodul in das zu steuernde medizinische Gerät integriert sind. Aufgrund der erfindungsgemäß erreichten einfachen freien Koppelbarkeit eines Fußschalters mit einem Receiver ist die aus dem Stand der Technik bekannte externe Anordnung von Receivern, denen ein Fußschalter praktisch fest zugeordnet ist und die mit verschiedenen medizinischen Geräten verbunden werden können, nicht mehr erforderlich. Das NFC-Lesemodul kann dabei räumlich getrennt vom eigentlichen Receiver in dem medizinischen Gerät integriert sein, um ein einfacherer Koppeln zu ermöglichen. Beispielsweise kann der NFC-Receiver an der Frontseite des medizinischen Gerätes angeordnet sein, während der Receiver an einer beliebigen anderen Stelle innerhalb des Geräts anordenbar ist.

Es ist bevorzugt, wenn der Transponder ein, vorzugsweise passiver, RFID-Transponder ist. Entsprechende Transponder benötigen keine eigene Energiequelle, sondern erhalten die zum Auslesen der darauf abgelegten Informationen benötigte Energie aus dem elektromagnetischen Feld im Nahbereich eines NFC-Lesemoduls.

Bei dem zu steuernden medizinischen Gerät handelt es sich vorzugsweise um einen HF-Generator, insbesondere für die Endoskopie, einen Ultraschall-Generator und/oder ein Lasersystem.

Auch wenn sich die Erläuterungen des erfindungsgemäßen Systems grundsätzlich auf die Kopplung eines Fußschalters und eines Receivers beziehen, ist unmittelbar ersichtlich, dass bei dem erfindungsgemäßen System eine freie Kopplung eines beliebigen von mehreren Fußschalterm mit einem beliebigen von mehreren Receivern möglich ist.

Zur Erläuterung des erfindungsgemäßen Verfahrens wird auf die vorstehenden Ausführungen verwiesen.

Die Erfindung wird nun anhand einer vorteilhaften Ausführungsform unter Bezugnahme auf die beigefügten schematischen Zeichnungen beispielhaft beschrieben. Es zeigen:
- Figur 1:: ein Ausführungsbeispiel eines erfindungsgemäßen Systems; und
- Figur 2:: das System aus Figur 1 während des Koppelns.

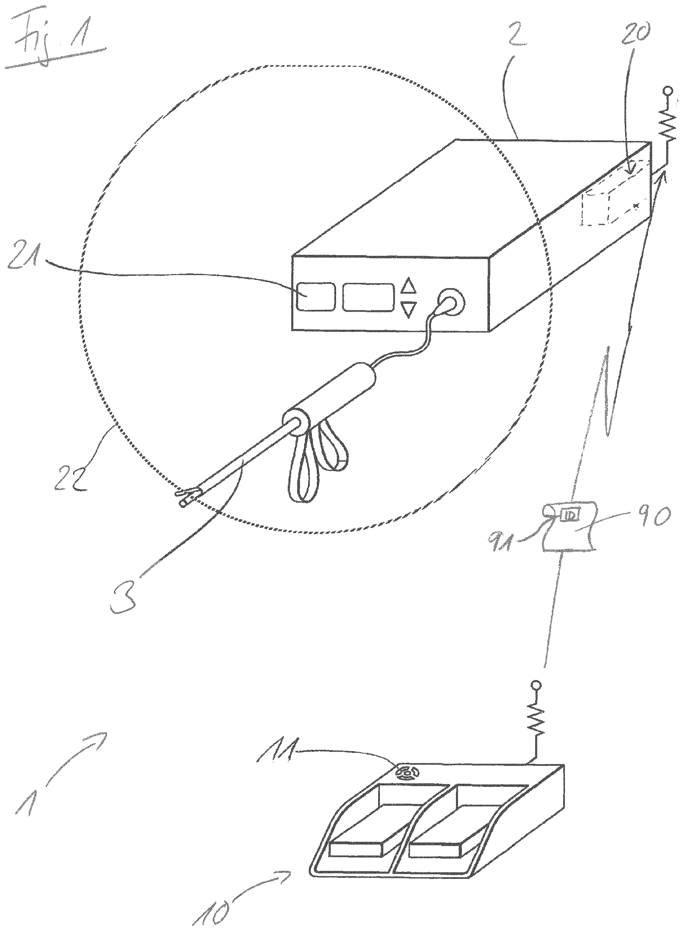

In Figur 1 ist ein erfindungsgemäßes System 1 dargestellt, bei dem ein medizintechnisches Gerät 2, nämlich ein HF-Generator 2 für ein endoskopisches Resektionswerkzeug 3, über einen Fußschalter 10 gesteuert wird.

Der Fußschalter 10 ist dabei drahtlos mit dem medizinischen Gerät 2 verbunden. Dazu weist das medizinische Gerät 2 einen integrierten Receiver 20 auf, der zum Empfang von dem Fußschalter 10 ausgesendete Steuerungssignale 90 ausgebildet ist. Die vom Receiver 20 empfangenen Steuerungssignale 90 werden vom Receiver 20 oder einer anderen Komponente des medizinischen Geräts 2 nach einer positiven Prüfung unmittelbar vom medizinischen Gerät 2 umgesetzt.

Die Steuerungssignale 90 umfassen neben den eigentlichen Steuerungsbefehlen auch eine eindeutige Identifikation 91, über welche die Steuerungssignale 90 eindeutig einem bestimmten, nämlich den in Figur 1 gezeigten Fußschalter 10 zugeordnet werden können. Die Prüfung der vom Receiver 20 empfangenen Steuerungssignale 90 umfasst die Prüfung der in den Steuerungssignalen 90 enthaltenen Identifikation 91. Es sollen dabei nur die Steuerungsbefehle solcher Steuerungssignale 90 verarbeitet und letztendlich umgesetzt werden, die von einem vorgegebenen Fußschalter 10 stammen. Eine solche 1-zu-1-Zuordnung eines bestimmten Fußschalters 10 zu einem medizinischen Gerät 2 bzw. dessen Receiver 20 erfolgt im Rahmen des Koppelns von Fußschalter 10 und Receiver 20.

Für eben dieses Koppeln umfasst der Receiver 20 ein NFC-Lesemodul 21. Auch wenn das NFC-Lesemodul im dargestellten Ausführungsbeispiel räumlich getrennt von dem Receiver 20 angeordnet ist, ist durch Integration sowohl des Receivers 20 als auch des NFC-Lesemoduls 21 in dasselbe medizinische Gerät 2 eine eindeutige Zuordnung zwischen NFC-Lesemodul 21 und Receiver 20 gegeben, sodass das NFC-Lesemodul 21 als zum Receiver 20 gehörig angesehen werden kann.

Das NFC-Lesemodul 21 ist dazu ausgebildet, Informationen von einem innerhalb des Nahbereichs 22 mit einem Radius von ca. 10 cm um das NFC-Lesemodul 21 befindlichen, geeigneten Transponder 11 auszulesen. Im Normalzustand ist das NFC-Lesemodul 21 deaktiviert, kann aber zum Koppeln durch einen Nutzer für einen kurzen Zeitraum von ca. 10 Sekunden aktiviert werden.

Am Fußschalter 11 ist ein vom NFC-Lesemodul 21 grundsätzlich auslesbarer Transponder 11 angeordnet. Bei dem Transponder 11 handelt es sich um einen passiven RFID-Transponder, indem zumindest die Identifikation 91 des Fußschalters 10 hinterlegt ist.

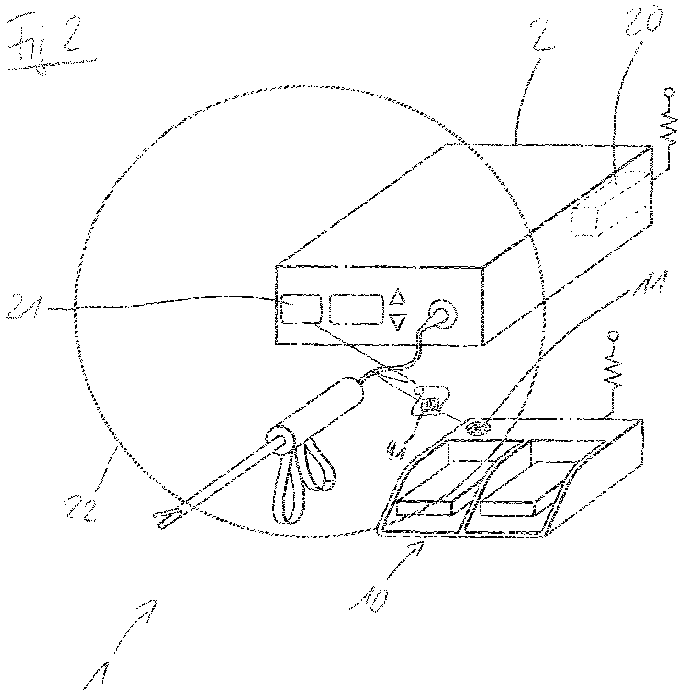

Zum Koppeln des Fußschalters 10 mit dem Receiver 20 bzw. dem medizinischen Gerät 2 wird das NFC-Lesemodul 21 aktiviert und der Fußschalter 10 - wie in Figur 2 dargestellt - so in die Nähe des medizinischen Gerätes 2 gebracht, dass sich der Transponder 11 des Fußschalters 10 im Nahbereich 22 des NFC-Lesemoduls 21 befindet. Es wird dann abgewartet, bis das Lesemodul 21 dann sämtliche Informationen vom Transponder 11 ausgelesen hat, worin zumindest die eindeutige Identifikation 91 des Fußschalters 10 enthalten ist.

Im dargestellten Ausführungsbeispiel ist die Kopplung von Fußschalter 10 und Receiver 20 bzw. dem medizinischen Gerät 2 durch das Auslesen der eindeutigen Identifikation 91 des Fußschalters 10 durch das NFC-Lesemodul 21 bereits abgeschlossen. Ab dem Zeitpunkt dieses Auslesens verarbeitet der Receiver 20 bzw. das medizinische Gerät 2 ausschließlich empfangene Steuerungssignale 90, welche eben diese eindeutige Identifikation 91 enthalten (vgl. Figur 1).

Es ist aber auch möglich, dass die ausgelesenen Informationen verwendet werden, um eine initiale drahtlose bidirektionale Verbindung zwischen Receiver 20 und Fußschalter 10 aufzubauen, über die dann noch weitere Informationen, wie bspw. Verschlüsselungsinformationen, ausgetauscht werden, bevor die Kopplung tatsächlich abgeschlossen ist und Steuerungssignale 90, wie beschrieben, verarbeitet werden können. Diese zusätzlichen Informationen können alternativ auch über eine NFC-Anordnung ausgetauscht werden, bei der auch der Fußschalter 10 über ein NFC-Lesemodul verfügt, das dann einen dem Receiver 20 zugeordneten Transponder auslesen kann.

## Patentansprüche

1. System (1) umfassend einen Fußschalter (10) zur Steuerung eines medizintechnischen Geräts (2) und einen Receiver für das zu steuernde medizintechnische Gerät (2) (20) als Komponenten (10, 20), wobei Fußschalter (10) und Receiver (20) zur drahtlosen Übermittlung von eindeutig zuordenbaren Steuerungssignalen (90) nach erfolgter Kopplung wenigstens vom Fußschalter (10) zum Receiver (20) ausgebildet sind, **dadurch gekennzeichnet, dass**
eine der Komponenten (20, 10) ein NFC-Lesemodul (21) zum Auslesen eines Transponders (11) im Nahbereich (22) und die andere Komponente (10, 20) einen vom NFC-Lesemodul (21) auslesbaren Transponder (11) umfasst, wobei die Komponente (20, 10) umfassend das NFC-Lesemodul (12) dazu ausgebildet ist, die Kopplung von Fußschalter (10) und Receiver (20) auf Basis von vom Transponder (11) ausgelesenen Informationen wenigstens zu initiieren.

2. System nach Anspruch 1,
**dadurch gekennzeichnet, dass**
beide Komponenten (10, 20) jeweils ein NFC-Lesemodul (21) und einen Transponder (11) aufweisen und dazu ausgebildet sind, die Kopplung durch wechselseitig von den Transpondern (11) ausgelesene Informationen wenigstens zu initiieren.

3. System nach Anspruch 1 oder 2,
**dadurch gekennzeichnet, dass**
die vom Transponder (11) ausgelesenen Informationen ausreichend für eine unmittelbare und abschließende Kopplung der Komponenten (10, 20) sind.

4. System nach einem der vorhergehenden Ansprüche,
**dadurch gekennzeichnet, dass**
Fußschalter (10) und Receiver (20) dazu ausgebildet sind, eine Kopplung nach Auslesen der Informationen vom Transponder (11) durch Datenaustausch über die drahtlose Verbindung zwischen Fußschalter (10) und Receiver (20) durchzuführen und/oder zu verifizieren.

5. System nach einem der vorhergehenden Ansprüche,
**dadurch gekennzeichnet, dass**
der Receiver (20) ein NFC-Lesemodul (21) zum Auslesen eines Transponders (11) im Nahbereich und der Fußschalter (10) einen vom NFC-Lesemodul (20) auslesbaren Transponder (11) aufweist.

6. System nach einem der vorhergehenden Ansprüche,
**dadurch gekennzeichnet, dass**
der Nahbereich (22) einen maximalen Abstand zwischen Transponder (11) und NFC-Lesegerät (21) von 10 cm, vorzugsweise von 5 cm umfasst.

7. System nach einem der vorhergehenden Ansprüche,
**dadurch gekennzeichnet, dass**
das oder die NFC-Lesemodule (21) im Grundzustand deaktiviert und zur Kopplung aktivierbar sind.

8. System nach einem der vorhergehenden Ansprüche,
**dadurch gekennzeichnet, dass**
der Receiver (20) und/oder ein NFC-Lesemodul (21) in das zu steuernde medizinische Gerät (2) integriert ist.

9. System nach einem der vorhergehenden Ansprüche,
**dadurch gekennzeichnet, dass**
der Transponder (11) ein, vorzugsweise passiver, RFID-Transponder ist.

10. System nach einem der vorhergehenden Ansprüche,
**dadurch gekennzeichnet, dass**
das zu steuernde medizintechnische Gerät (2) ein HF-Generator, insbesondere für die Endoskopie, ein Ultraschall-Generator und/oder ein Lasersystem ist.

11. Verfahren zur Kopplung eines Fußschalters (10) zur Steuerung eines medizintechnischen Geräts (2) und einen an dem zu steuernden medizintechnischen Gerät (2) angeschlossenen Receiver (20) gemäß dem System (1) nach einem der vorhergehenden Ansprüche, mit den Schritten:
a) Initiieren der Kopplung zwischen Fußschalter (10) und Receiver (20) durch Heranführen des Fußschalters (10) an den Receiver (20), sodass sich der Transponder (11) der einen Komponente (10, 20) im Nahbereich des NFC-Lesemoduls (21) der anderen Komponente (20, 10) befindet; und
b) Abwarten bis die zur Kopplung erforderlichen Informationen ausgetauscht sind und die Kopplung abgeschlossen ist.
